# EUROPEAN PATENT SPECIFICATION

(11) **EP 0 530 986 B1**
(45) Date of publication and mention of the grant of the patent: **09.06.1999**
(21) Application number: 92307228.4
(22) Date of filing: 07.08.1992
(51) Int. Cl.: A01N 43/80, A01N 25/22, C07D 275/03

(54) **Nitrogen-based stabilizers for 3-isothiazolones**
Stickstoffenthaltende Stabilisatoren für 3-Isothiazolone
Stabilisateurs à base d'azote pour des 3-isothiazolones

(30) Priority: 13.08.1991 GB 9117448
(43) Date of publication of application: 10.03.1993
(73) Proprietor: ROHM AND HAAS COMPANY, Philadelphia Pennsylvania 19105 (US)
(72) Inventor: Reeve, Paul Francis David#, Le Plan de Grasse# (FR)
(74) Representative: Buckley, Guy Julian

(56) References cited:
- EP-A- 0 332 336
- EP-A- 0 443 821
- GB-A- 2 200 846
- US-A- 4 067 878
- US-A- 4 173 643
- US-A- 4 396 413
- JAPANESE PATENTS GAZETTE Section Ch, Week 35, 11 October 1978 Derwent Publications Ltd., London, GB; Class C, AN 78-62762A/35
- CHEMICAL PATENTS INDEX, DOCUMENTATION ABSTRACTS JOURNAL Section Ch, Week 9042, 12 December 1990, Derwent Publications Ltd., London, GB; Class C, AN 90-318326/42
- CHEMICAL ABSTRACTS, vol. 79, no. 13, 1 October 1973, Columbus, OH (US); p. 159, no. 74918h

## Description

This invention concerns the stabilization of 3-isothiazolone compounds, particularly in metal working fluid concentrates, by the incorporation with those compounds of certain nitrogen-based compounds.

Isothiazolones are of significant commercial value as microbicides to prevent spoilage of certain aqueous and non-aqueous products caused by microorganisms. They are highly effective microbicides (as used herein, "microbicides" includes bactericides, fungicides and algicides, and microbicidal activity is intended to include both the elimination of and the inhibition or prevention of growth of microbial organisms such as bacteria, fungi and algae). By suitable choice of functional groups, they are useful in a broad range of applications.

One significant area of application for isothiazolones is as microbicides in metal working fluids. Metal working fluids are proprietary combinations of chemicals which may contain, inter alia, ingredients such as alkanolamines, petroleum sulfonate surfactants, oils (naphthenic, paraffinic, etc), chlorinated paraffins and fatty esters, sulfurized fatty compounds, phosphate esters, fatty acids and their amine salts, glycols, polyglycols, boric acid esters and amides. They are utilized in the milling, machining, drilling, and other processing technologies for fabricating metal for the purposes of lubricating, cooling, preventing surface corrosion, and the like. They are sold in the form of active metal working fluid (MWF) concentrates, and are diluted in use to 1-10% active ingredient in water.

Because metal working fluids are recycled and stored, the growth of micro-organisms is favoured. Isothiazolones have been found effective in preventing the growth of such organisms. However, certain components in the metal working fluids tend to destroy the isothiazolone and so remove its microbicidal protective activity. This is a particular problem when the MWFs are in concentrate form. It has been found that even some other microbicides, present in combination with isothiazolones, may attack the isothiazolones. An example of this is the sodium salt of 2-mercapto pyridine-N-oxide (sodium omadine), which has been found to remove 5-chloro-2-methyl isothiazolone from any system in which the two are present together.

Indeed, generally, it has long been recognized that either in storage prior to addition to a substrate to be treated or after addition, the efficacy of isothiazolones in many environments may be decreased because they are not stable under practical conditions of long term storage. Means have thus been sought for some time to improve the stability of isothiazolones.

US-A-3,870,795 and 4,067,878 teach the stabilization of isothiazolones against chemical decomposition by addition of a metal nitrite or metal nitrate, but teach that other common metal salts, including carbonates, sulfates, chlorates, perchlorates, and chlorides are ineffective in stabilizing solutions of isothiazolones, such solutions usually being in water or in an hydroxylic solvent. US-A-4,150,026 and US-A-4,241,214 teach that metal salt complexes of isothiazolones are useful because they have enhanced thermal stability, while retaining biological activity.

It is known to use certain organic stabilizers for isothiazolones, generally for use situations where metal salts may create problems, such as corrosion, coagulation of latices, insolubility in non-aqueous media, interaction with the substrate to be stabilized, and the like. Formaldehyde or formaldehyde-releasing chemicals are known as stabilizers, (see US-A-4,165,318 and 4,129,448), as are certain organic chemicals such as orthoesters (EP-A-315,464), epoxides (EP-A-342,852), and carbonyl compounds (EP-A-435439).

US-A-4396413 and JP78-62762A disclose compositions containing isothiazolones and, respectively, pyridine/pyrimidine and pyridine. However, there is no mention of any stabilising effect against chemical degradation. JP-90-318326A discloses compositions comprising isothiazolone and DMF or N-methyl-2-pyrrolidone. There is no disclosure of use in metal working fluid concentrates.

Our copending European Application No. 91301324.9 discloses stabilizers for isothiazolones characterized in that they are sulphur-containing, and are capable of reversibly associating with isothiazolones.

We have now discovered a further class of compounds which provide considerable and surprising stability to isothiazolones against decomposition, particularly in MWF concentrates. In its broadest aspect the invention provides a method of protecting against chemical degradation in a metal-working fluid concentrate an isothiazolone of the formula: 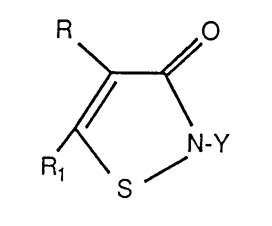 wherein
Y is (C₁-C₁₈)alkyl or (C₃-C₁₂)cycloalkyl each optionally substituted with one or more of hydroxy, halo, cyano, alkylamino, dialkylamine, arylamino, carboxy, carbalkoxy, alkoxy, aryloxy, alkylthio, arylthio, haloalkoxy, cycloaklylamino, carbamoxy, or isothiazolonyl; an unsubstituted or halo-substituted (C₂-C₈) alkenyl or alkynyl; a (C₇-C₁₀)aralkyl optionally substituted with one or more of halogen, (C₁-C₄)alkyl or (C₁-C₄)alkoxy; or an aryl optionally substituted with one or more of halogen, nitro, (C₁-C₄)alkyl, (C₁-C₄)alkyl-acylamino, carb(C₁-C₄)alkoxy or sulfamyl; and
R and R¹ are each independently H, halogen, (C₁-C₄) alkyl, (C₄-C₈) cycloalkyl or joined together to form a phenyl; comprising incorporating in the metal-working fluid concentrate therewith an effective amount of pyridine, pyridine-N-oxide, 2-pyrrolidone, 1-methyl-2-pyrrolidone, s-triazine or dimethyl oxime. The precise nature of the association between the nitrogen containing compounds of the present invention and isothiazolones is not yet fully understood. It is believed that hydrogen-bonding may play a role, although the invention is of course not limited to such a case. The entity resulting from this association is hereinafter referred to as the 'association product'.

The association product is generally hydrolysable, and thus the association process may be reversed by hydrolysis, usually accomplished through dilution.

The invention provides a particularly advantageous form of protection, or stabilization. These stabilizers can be used to 'lock up' an isothiazolone, in the form of a stable association product which is resistant to chemical degradation, and then when desired they can be made to 'release' the isothiazolone by reversing the process of association - usually simply by dilution of the product.

In another aspect the invention provides a composition comprising a composition comprising a metal-working fluid concentrate, an isothiazolone as defined in any preceding claim, pyridine, pyridine-N-oxide, 2-pyrrolidone, a-methyl-2-pyrrolidone, s-triazine or dimethyl oxime, and optionally a solvent.

The invention has particular value in the field of metal working fluids, which are commonly stored in concentrate form. Some of the components in MWFs are extremely aggressive towards isothiazolones when in concentrate form, but of little threat when diluted to the normal use dilution. Thus the present invention can be employed by adding a stabilizer to protect the isothiazolones in the concentrate, the isothiazolone then being released automatically upon dilution by hydrolysis of the isothiazolone-stabilizer association.

The isothiazolones which are stabilized include those disclosed in US-A-3,523,121 and 3,761,488 and represented by the formula: 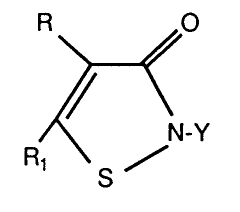 as defined above.

Preferred substituents for Y are substituted or unsubstituted (C₁-C₁₈) alkyl or (C₃-C₁₂) cycloalkyl; R is preferred to be H, or Me; and R¹ is preferred to be H. Representative of such preferred Y substituents are methyl, ethyl, propyl, isopropyl, butyl, hexyl, octyl, cyclohexyl, benzyl, 3,4-dichlorobenzyl, 4-methoxybenzyl, 4-chlorobenzyl, 3,4-dichlorophenyl, 4-methoxyphenyl, hydroxymethyl, chloromethyl, chloropropyl, hydrogen, and the like.

Particularly preferred isothiazolones are 2-methyl-3-isothiazolone, and 2-n-octyl-3-isothiazolone.

In order for protection of the isothiazolone to be effective, the molar ratio of stabilizer to isothiazolone should preferably be at least 0.1:1, and most preferably at least 0.5:1. The minimum ratio generally depends on the aggressiveness of the system in which the isothiazolone is contained. A typical preferred range is from 0.5:1 to 1.5:1. However, some of the compounds which act as stabilizers may find other uses in the systems to which isothiazolones have been added - eg, as microbicides in their own right. In such cases, stabilizer: isothiazolone molar ratios may be greater than 10:1.

Compositions of isothiazolone and stabilizer may additionally contain solvents. A suitable solvent will be any organic solvent which dissolves the isothiazolone, is compatible with the proposed end use, does not destabilize the isothiazolone, and does not react with the stabilizer to prevent its protective action.

Hydroxylic solvents, for example, polyols, such as glycols, alcohols and the like, may be used. In certain formulations, hydrocarbons, either aliphatic or aromatic, are useful solvents.

Preferred solvents are capped polyols, wherein the free hydroxyl group is replaced with an ether or ester function. Especially preferred are 2,5,8,11-tetraoxadodecane, commonly known as triethylene glycol dimethyl ether, and 4,7-dioxaundecanol-1 acetate, commonly known as diethylene glycol butyl ether acetate.

Water is a solvent for certain of the preferred isothiazolones and, the stabilizer may be employed in aqueous formulations.

In certain cases the association products formed according to the invention may be in the form of a solid precipitate. This can generally be avoided by standard techniques for increasing the solubility product of a system such as adding emulsifiers, or diluting the system. Those skilled in the art will have little difficulty in altering the conditions to avoid precipitate formation if that should be a problem in any particular case.

It is known in the art that the performance of microbicides may be enhanced by combination with one or more other microbicides. Thus, other known microbicides may be combined advantageously with the compositions or compounds of this invention.

Uses of these protected isothiazolones may be at any locus subject to contamination by bacteria, fungi, yeast or algae. Typical loci are in aqueous systems such as water cooling, laundry rinse water, oil systems such as cutting oils, oil fields and the like where microorganisms need to be killed or where their growth needs to be controlled. However, they may also be used in all applications for which known microbicidal compositions are useful; preferred utilities of the compositions are to protect wood paint, adhesive, glue, paper, textile, leather, plastics, cardboard, lubricants, cosmetics, food, caulking, feed and industrial cooling water from microorganisms.

The following Examples are intended to illustrate the present invention. All percentages are by weight unless otherwise specified. Methods for quantitative determination of the isothiazolones in the following Examples in metal working fluids are described in detail in "Kathon® 886 MW Microbicide and Kathon® 893MW Fungicide: Analysis in Metalworking Fluids by High-Performance Liquid Chromatography", 1988, Rohm and Haas Company.

### EXAMPLE 1

This Example illustrates the protection afforded to isothiazolones by the stabilizers of the present invention.

Monoethanolamine is known to degrade most isothiazolones when in contact with them in concentrated form. It is a component of metal working fluids, and is therefore a particular problem when present with isothiazolones in MWF concentrates.

In the following Example, a test system was used which comprised a 1:1 water/propylene glycol solvent, 10% monoethanolamine, 900ppm of 2n-octyl-3-isothiazolone, and a stabilizer according to the invention. The system was maintained at 25°C, and the concentration of isothiazolone remaining after 1,2,4,8 and 12 weeks respectively was determined. Isothiazolone concentration was determined by removing an aliquot from the system at the appropriate time, diluting it 50-fold with a 1:1 water/propylene glycol solution, and then analysing for isothiazolone by HPLC.

Referring to Table 1, it should be noted that the initial concentration of isothiazolone in each case was 900ppm. The subsequent readings are percentages of that value, and are accurate to ±5%.

**TABLE 1**

| STABILIZER | STABILIZER CONCENTRATION | % ISOTHIAZOLONE REMAINING (±5%) | | | | |
|---|---|---|---|---|---|---|
| | (PPM) | 1WK | 2WKS | 4WKS | 8WKS | 12WKS |
| None (control) | 0 | 72 | 0 | | | |
| Pyridine | 1000 | 98 | 98 | 98 | 100 | 100 |
| 2-pyrrolidone | 1000 | 97 | 98 | 94 | 94 | 97 |
| 1-methyl-2-pyrrolidone | 1000 | 97 | 96 | 91 | 96 | 98 |
| Dimethyl oxime | 1000 | - | 94 | 92 | 96 | 96 |
| Pyridine-N-oxide | 1000 | 91 | 83 | 67 | 48(6wks) | |
| s-triazine | 1000 | 98 | 82 | 59 | 18 | |

Referring to Table 1, it can be seen that with no stabilizer present the isothiazolone is rapidly decomposed by the monoethanolamine, having completely disappeared within two weeks. The present invention is limited to those nitrogen-containing, sulphur-free compounds which are capable of reversibly associating with the isothiazolone. As can be seen from the remaining examples in Table 1, these provide exceptional protection for the isothiazolone. As a general rule, it is considered that useful stabilization is achieved if approximately 80% of the isothiazolone remains after 4 weeks, although when compared with the loss of isothiazolone with no stabilizer present, retention of about 60% isothiazolone after 4 weeks may be considered to be effective stabilization.

## Claims

1. A method of protecting against chemical degradation in a metal-working fluid concentrate an isothiazolone of the formula: 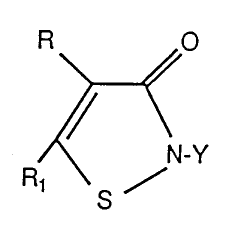 wherein
Y is (C₁-C₁₈)alkyl or (C₃-C₁₂)cycloalkyl each optionally substituted with one or more of hydroxy, halo, cyano, alkylamino, dialkylamine, arylamino, carboxy, carbalkoxy, alkoxy, aryloxy, alkylthio, arylthio, haloalkoxy, cycloaklylamino, carbamoxy, or isothiazolonyl; an unsubstituted or halo-substituted (C₂-C₈) alkenyl or alkynyl; a (C₇-C₁₀)aralkyl optionally substituted with one or more of halogen, (C₁-C₄)alkyl or (C₁-C₄)alkoxy; or an aryl optionally substituted with one or more of halogen, nitro, (C₁-C₄)alkyl, (C₁-C₄)alkyl-acylamino, carb(C₁ -C₄)alkoxy or sulfamyl; and
R and R¹ are each independently H, halogen, (C₁-C₄) alkyl, (C₄-C₈) cycloalkyl or joined together to form a phenyl; comprising adding to the metal-working fluid concentrate an effective amount of pyridine, pyridine-N-oxide, 2-pyrrolidone, 1-methyl-2-pyrrolidone, s-triazine or dimethyl oxime.

2. Method according to claim 1, wherein Y is (C₁-C₁₈) alkyl or (C₃-C₁₂) cycloalkyl; R is H or Me; and R¹ is H.

3. Method according to claim 2 wherein the isothiazolone is 2-methyl-3-isothiazolone or 2n-octyl-3-isothiazolone.

4. Composition comprising a metal-working fluid concentrate, an isothiazolone as defined in any preceding claim, pyridine, pyridine-N-oxide, 2-pyrrolidone, 1-methyl-2-pyrrolidone, s-triazine or dimethyl oxime, and optionally a solvent.

5. Method or composition according to any preceding claim wherein the molar ratio of pyridine, pyridine-N-oxide, 2-pyrrolidone, 1-methyl-2-pyrrolidone, s-triazine or dimethyl oxime to isothiazolone is at least 0.1:1, preferably at least 0.5:1.

6. Use of pyridine, pyridine-N-oxide, 2-pyrrolidone, 1-methyl-2-pyrrolidone, s-triazine or dimethyl oxime to protect an isothiazolone against chemical degradation in metal-working fluid concentrates.

## Patentansprüche

1. Verfahren zum Schutz eines Isothiazolons der Formel 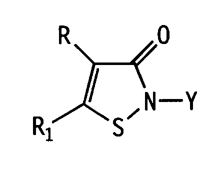 vor chemischer Zersetzung in einem Konzentrat einer Metallbearbeitungsflüssigkeit, worin Y gleich C₁- bis C₁₈-Alkyl oder C₃- bis C₁₂-Cycloalkyl ist, die jeweils wahlweise mit einem oder mehreren Hydroxy, Halo, Cyano, Alkylamino, Dialkylamino, Arylamino, Carboxy, Carbalkoxy, Alkoxy, Aryloxy, Alkylthio, Arylthio, Halogenalkoxy, Cycloalkylamino, Carbamoxy oder Isothiazolonyl substituiert sind, ein unsubstituiertes oder halogensubstituiertes C₂- bis C₈-Alkenyl oder -Alkinyl, ein C₇- bis C₁₀-Aralkyl, das wahlweise mit einem oder mehreren Halogen substituiert ist, C₁- bis C₄-Alkyl oder C₁- bis C₄-Alkoxy oder ein Aryl, das wahlweise mit einem oder mehreren Halogen, Nitro, C₁- bis C₄-Alkyl, C₁- bis C₄-Alkylacylamino, Carb(C₁-C₄)alkoxy oder Sulfamyl substituiert ist, und R und R¹ unabhängig voneinander H, Halogen, C₁- bis C₄-Alkyl, C₄- bis C₈-Cycloalkyl sind oder zusammen einen Phenylring bilden, umfassend Zugeben einer wirksamen Menge von Pyridin, Pyridin-N-oxid, 2-Pyrrolidon, 1-Methyl-2-pyrrolidon, s-Triazin oder Dimethyloxim zu dem Konzentrat der Metallbearbeitungsflüssigkeit.

2. Verfahren nach Anspruch 1, wobei Y gleich C₁- bis C₁₈-Alkyl oder C₃-bis C₁₂-Cycloalkyl ist, R gleich H oder Me ist und R¹ gleich H ist.

3. Verfahren nach Anspruch 2, wobei das Isothiazolon 2-Methyl-3-isothiazolon oder 2-n-Octyl-3-isothiazolon ist.

4. Zusammensetzung enthaltend ein Konzentrat einer Metallbearbeitungsflüssigkeit, ein Isothiazolon wie in einem der vorstehenden Ansprüche definiert, Pyridin, Pyridin-N-oxid, 2-Pyrrolidon, 1-Methyl-2-pyrrolidon, s-Triazin oder Dimethyloxim und wahlweise ein Lösungsmittel.

5. Verfahren oder Zusammensetzung nach einem der vorstehenden Ansprüche, wobei das molare Verhältnis von Pyridin, Pyridin-N-oxid, 2-Pyrrolidon, 1-Methyl-2-pyrrolidon, s-Triazin oder Dimethyloxim zu Isothiazolon wenigstens 0,1:1, vorzugsweise wenigstens 0,5:1 beträgt.

6. Verwendung von Pyridin, Pyridin-N-oxid, 2-Pyrrolidon, 1-Methyl-2-pyrrolidon, s-Triazin oder Dimethyloxim, um ein Isothiazolon vor chemischer Zersetzung in einem Konzentrat einer Metallbearbeitungsflüssigkeit zu schützen.

## Revendications

1. Procédé de protection contre la dégradation chimique, dans un concentré fluide pour le travail des inétaux, d'une isothiazolone de formule : 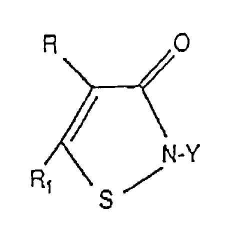 dans laquelle
Y est un groupe alkyle en C₁ à C₁₈ Ou cycloalkyle en C₃ a C₁₂, chacun étant facultativement substitué par un ou plusieurs parmi les groupes hydroxy, halogéno, cyano, alkylamino, dialkylamino, arylamino, carboxy, carbalcoxy, alcoxy, aryloxy, alkylthio, arylthio, halogénoalcoxy, cycloalkylamino, carbamoxy ou isothiazolonyle ; un groupe alcényle ou alcynyle en C₂ à C₈, non substitué ou substitué par un halogène ; un groupe aralkyle en C₇ à C₁₀ facultativement substitué par un ou plusieurs ingrédients parmi un halogène, un groupe alkyle en C₁ à C₄ ou alcoxy en C₁ à C₄ ; ou un groupe aryle facultativement substitué par un ou plusieurs ingrédients parmi un halogène, un groupe nitro, alkyle en C₁ à C₄, alkyl en C₁ à C₄-acylamino, carb-alcoxy en C₁ à C₄ ou sulfamyle ; et R et R¹ sont chacun indépendamment H, un halogène, un groupe alkyle en C₁ à C₄, cycloalkyle en C₄ à C₈ ou sont joints ensemble pour former un groupe phényle ; comprenant l'addition au concentré fluide peur le travail des métaux d'une quantité efficace de pyridine, de pyridine-N-oxyde, de 2-pyrrolidone, de 1-méthyl-2-pyrrolidone, de s-triazine ou de diméthyloxime .

2. Procédé selon la revendication 1, dans lequel Y est un groupe alkyleen C₁ à C₁₈ ou cycloalkyle en C₃ à C₁₂ ; R est H ou Me ; et R¹ est H.

3. Procédé selon la revendication 2, dans lequel l'isothiazolone est la 2-méthyl-3-isothiazolone ou la 2n-octyl-3-isothiazolone.

4. Composition comprenant un concentré fluide pour le travail des métaux, une isothiazolone telle que définie dans l'une quelconque des revendications précédentes, de la pyridine, de la pyridine-N-oxyde, de la 2-pyrrolidone, de la 1-méthyl-2-pyrrolidone, de la s-triazine ou de la diméthyloxime et, facultativement, un solvant.

5. Procédé ou composition selon l'une quelconque des revendications précédentes, où le rapport molaire de pyridine, pyridine-N-oxyde, 2-pyrrolidone, 1-méthyl-2-pyrrolidone, s-triazine ou diméthyloxime à l'isothiazolone est d'au moins 0,1:1, de préférence au moins 0,5:1.

6. Utilisation de pyridine, de pyridine-N-oxyde, de 2-pyrrolidone, de 1-méthyl-2-pyrrolidone, de s-triazin ou de diméthyloxime pour protéger une isothiazolone contre la dégradation chimique dans des concentrés fluides pour le travail des métaux.
